(19) Europäisches Patentamt

European Patent Office

Office européen des brevets

(11) Veröffentlichungsnummer: **0 145 641**

**B1**

# (12) EUROPÄISCHE PATENTSCHRIFT

(45) Veröffentlichungstag der Patentschrift:
23.09.87

(51) Int. Cl.⁴: **A 61 F 2/30**

(21) Anmeldenummer: **84730121.5**

(22) Anmeldetag: **06.11.84**

(54) **Bausatz für eine Resektionsprothese.**

(30) Priorität: **08.11.83 DE 3340767**

(43) Veröffentlichungstag der Anmeldung:
**19.06.85 Patentblatt 85/25**

(45) Bekanntmachung des Hinweises auf die Patenterteilung:
**23.09.87 Patentblatt 87/39**

(84) Benannte Vertragsstaaten:
**AT BE CH DE FR GB IT LI**

(56) Entgegenhaltungen:
**EP-A-0 011 665**
**DE-A-3 205 577**

(73) Patentinhaber: **MECRON MEDIZINISCHE PRODUKTE GMBH, Nunsdorfer Ring 25- 27, D-1000 Berlin 48 (DE)**

(72) Erfinder: **Anapliotis, Emmanuel, Tollensestrasse 16, D-1000 Berlin 37 (DE)**
Erfinder: **Kranz, Curt, Landshuter Strasse 5, D-1000 Berlin 30 (DE)**

(74) Vertreter: **Christiansen, Henning, Dipl.- Ing., Dietrich- Schäfer- Weg 21, D-1000 Berlin 41 (DE)**

EP 0 145 641 B1

## Beschreibung

Die Erfindung betrifft einen Bausatz der im Oberbegriff des Anspruchs 1 angegebenen Art.

Ein solcher Bausatz ist bekannt aus der DE-A-32 05 577. Die dort beschriebenen Teilstücke, die zu einer Femurprothese gewünschter Länge zusammensteckbar sind, ergeben zusammengesetzt einen relativ starren, gerade verlaufenden Femurersatz. Nachteilig ist dabei, daß eine Demontage, welche im Falle einer Reoperation notwendig werden kann, nur unter Schwierigkeiten möglich ist.

Der mit der bekannten Prothese verbundene Nachteil, daß sich die einzelnen Teile nachträglich nur schlecht voneinander lösen lassen, führt gerade bei Reoperationen und einer im Zusammenhang damit notwendig werdenden Veränderung der Prothese zu Schwierigkeiten. Dabei ist es nämlich wünschenswert, daß die Prothesenteile durch eine einfache Handhabung wieder voneinander getrennt werden können, um gegebenenfalls durch Einsetzen anderer Teile eine veränderte Zusammenstellung zu ermöglichen.

Der in Anspruch 1 angegebenen Erfindung liegt demgemäß die Aufgabe zugrunde, einen Prothesenbausatz der eingangs genannten Gattung anzugeben, welcher es gestattet, die Prothesenteile ohne besonderen Kraftaufwand unter Benutzung eines einfachen Werkzeugs wieder voneinander zu lösen.

Besonders vorteilhaft bei der Erfindung ist die Tatsache, daß die Prothese beim Lösen der Teile nicht für Schraubbewegungen oder dergleichen verdreht werden muß, so daß die Demontage einzelner Teile auch bei noch teilweise implantiertem Prothesenschaft erfolgen kann.

Bei bevorzugten Weiterbildungen der Erfindung ist der zum Trennen vorgesehene Keil gabelförmig ausgestaltet, so daß der erforderliche Kraftangriff im Randbereich der Prothesenteile erfolgen kann und keine zum Inneren führenden Öffnungen erforderlich sind, welche durch Eindringen von Körperflüssigkeit das Lösen der Prothesenteile voneinander erschweren könnten. Gleichzeitig ist damit auch die Führung erleichtert und der rückwärtige Bereich des Keils läßt sich breiter ausgestalten, so daß er auch unter erschwerten Bedingungen leicht mit einem entsprechenden Schlagwerkzeug getroffen werden kann.

Günstig bei der Erfindung ist weiterhin, daß sich bei gabelförmiger Gestaltung des Keils die Ausnehmungen mit den Flächen, welche durch Wechselwirkung mit dem Keil ein Trennen benachbarter Elemente ermöglichen, derart in die Außenkontur der Prothese einfügen lassen, daß sie kaum störend in Erscheinung treten und vor allem die Funktionsfähigkeit der Prothese nicht beeinträchtigen. Wenn die Ausnehmungen durch Anfasungen gebildet werden, können diese bei einer entsprechend ausgebildeten Form des Nachbarteils derart übergriffen werden, daß gleichzeitig eine Sicherung gegen Verdrehen gegeben ist.

Einer vorteilhaften Weiterbildung der vorliegenden Erfindung liegt die Aufgabe zugrunde, einen Bausatz für eine Resektionsprothese insbesondere als Femurersatz zu schaffen, welcher im zusammengesetzten Zustand eine der Krümmung des natürlichen Oberschenkelknochens angenäherte Gestaltung und damit auch ein entsprechendes physiologisches Verhalten unter statischer und dynamischer Belastung zeigt. Dabei sollen möglichst viele Einzelteile des Bausatzes sowohl für den linken Femur als auch für den rechten geeignet sein, um die Lagerhaltung zu vereinfachen.

Diese weitere Aufgabe wird dadurch gelöst, daß die Längsachse des Zwischenteiles ähnlich dem Femur gekrümmt ist und die Ausformungen derart gestaltet sind, daß sie in zwei Stellungen ineinanderpassen, in welchen die aneinanderstoßenden Teile um einen Winkel um ihre Längsachse verdreht sind, der 180° beträgt oder dem natürlichen Winkel nahekommt, der zwischen den lokalen Krümmungsebenen der Längsachsen des Femur gelegen ist.

Andere vorteilhafte Weiterbildungen der Erfindung sind in den übrigen abhängigen Ansprüchen angegeben.

Anhand von in den Zeichnungen wiedergegebenen Ausführungsbeispielen wird das Prinzip der Erfindung und ihrer Ausführungsformen näher erläutert. Es zeigen:

Figur 1 ein Ausführungsbeispiel eines ersten Bausatzes einschließlich eines die Gelenkkugel tragenden Kopfteils,

Figuren 1a bis 1f Einzelheiten des Bausatzes gemäß Figur 1,

Figur 2 ein weiteres Ausführungsbeispiel eines Bausatzes, entsprechend Figur 1 sowie

Figuren 2a bis 2d Einzelheiten des Bausatzes gemäß Figur 2.

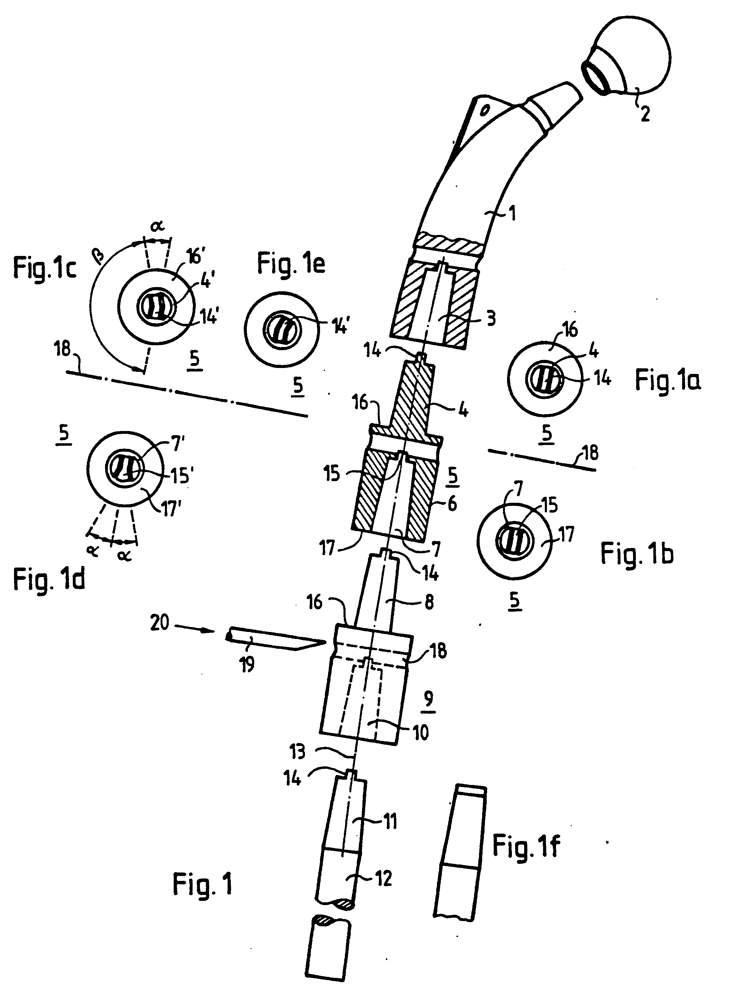

In Figur 1 ist für ein Kopfteil 1 einer Femurprothese eine aufsteckbare Gelenkkugel 2 vorgesehen. Am unteren Ende weist das Kopfteil 1 eine konische Bohrung 3 auf, in welche ein Zapfen 4 eines Zwischenteiles 5 mit kreiszylindrischem Rumpf 6 und einer konischen Bohrung 7 einsetzbar ist.

In die konische Bohrung 7 wiederum paßt ein Zapfen 8 eines weiteren Zwischenteiles 9, das im wesentlichen wie das Zwischenteil 5 aufgebaut ist, jedoch eine abweichende Länge aufweist. Mehrere solcher Zwischenteile 5, 9 können zu einem Schaft entsprechend der in Figur 1 gezeigten Resektionsprothese zusammengefügt werden. In das untere Ende des Schafts - nämlich in eine entsprechende konische Bohrung 10 des letzten Zwischenteiles 9 - ist ein konischer Zapfen 11 eines Endstückes 12 steckbar. Das Endstück 12 weist einen konischen Zapfen und einen zylindrischen Schaft mit gegenüber dem Zwischenteil verringertem Durchmesser auf, der dazu bestimmt ist, in den Markraum einzementiert zu werden.

Die Längsachse 13 der dargestellten Resektionsprothese ist ähnlich dem natürlichen Verlauf des Femur leicht gekrümmt. Bevorzugt ist diese Krümmung auf den Bereich der Zwischenteile 5, 9 konzentriert, während das Kopfteil 1 und das Endstück 12 im Schaftbereich im wesentlichen gerade verläuft. Durch die leichte Krümmung ergibt sich zusätzlich eine gewisse Elastizität, die der physiologischen besser entspricht. Auch ist die Krafteinleitung in den Femur verbessert.

Zur vereinfachten Darstellung sei hier angenommen, daß die Krümmungsebene der Längsachse 13 in der Zeichenebene gelegen ist. Bei längeren Prothesen kann die Krümmungsebene durchaus auch lokal unterschiedlich angeordnet sein - beispielsweise bei höher gelegenen Zwischenstücken in einer Ebene, wie sie der Zeichenebene entspricht. Bei anderen Ausführungen kann die Krümmungsebene auch mehr oder weniger zur Zeichenebene geneigt sein bzw. in unterschiedlichen Höhenabschnitten eine unterschiedliche Richtung aufweisen.

Da sich die geringe Krümmung über die gesamte Prothesenlänge von ca. 3 bis 4 Winkelgraden in ihrer Auswirkung auf die einzelnen Teile an diesen vielfach nur schwer erkennen läßt, muß sichergestellt sein, daß diese Zwischenteile in der Weise korrekt zusammengesetzt werden, daß sich der gewünschte Gesamtkrümmungsverlauf ergibt. Um auch eine Sicherung bezüglich der korrekten Richtung der Schaftkrümmung im Halsbereich zu bewirken, sind aufeinander abgestimmte (ineinander fügbare) Ausformungen als Sicherungen gegen Verdrehen um die Achse 13 vorgesehen, und zwar in Gestalt jeweils einer Feder 14 an den Zapfen 4, 8 und 11 und von Nuten 15 in den Bohrungen 3, 7 und 10. Die Federn und Nuten greifen im zusammengesetzten Zustand der Prothese ineinander.

Um das Zusammensetzen zu erleichtern, weisen diese Ausformungen gegeneinander ein geringes Spiel auf. Statt Federn und Nuten als Ausformungen können auch zwei Nasen mit zugehörigen Ausnehmungen oder dergl. vorgesehen sein. Diese Ausformungen können sowohl am Ende der Zapfen bzw. am Grund der Bohrungen, als auch an den aneinanderstoßenden Flächen 16 bzw. 17 vorgesehen sein.

Zu Figur 1 zeigt die Detaildarstellung gemäß Figur 1a eine Aufsicht auf das Zwischenteil 5, und zwar auf das Ende des Zapfens 4, der mit der Feder 14 versehen ist. In Figur 1b ist eine Aufsicht auf die andere Seite des Zwischenteiles 5 mit der Fläche 17 der konischen Bohrung 7 und der Nut 15 dargestellt. Wenn die Zeichnung um die Achse 18 gefaltet wird, ist ersichtlich, daß ein Zapfen 14 gemäß Figur 1a in eine Nut 15 gemäß Figur 1b paßt. Es ist ebenfalls ersichtlich, daß der Zapfen 14 auch dann in die Nut paßt, wenn das Zwischenteil 5 in Figur 1a um 180° um seine Längsachse gedreht ist. Hieraus folgt, daß die gekrümmten Zwischenteile 5, 9 nicht nur zum Aufbau einer linksseitigen Femurprothese geeignet sind, sondern in entsprechender Weise auch für den Aufbau einer rechtsseitigen Prothese verwendbar sind. Voraussetzung dabei ist, daß die Krümmungsebene einer rechtsseitigen Prothese um 180° gegenüber der Krümmungsebene für eine linksseitige Prothese um die Körpermittelachse geschwenkt ist.

Diese vereinfachende Voraussetzung trifft aber nicht über die ganze Länge eines Oberschenkelknochens zu. Vielmehr gibt es Bereiche im Verlaufe des Femur, wo zwischen der linksseitigen Krümmungsebene und der rechtsseitigen ein Winkel "beta" von weniger als 180° liegt. Hierfür sind die Ausführungsbeispiele geeignet, die in den Figuren 1c und 1d (in Anlehnung an die Figuren 1a und 1b) dargestellt sind. Die Bezugszeichen sind gegenüber den Figuren 1a und 1b mit einem Apostroph versehen. Aus Figur 1c geht hervor, daß die Feder 14' in dem stumpfen Winkel "beta" geknickt verläuft. Der Differenzwinkel zwischen 180° und "beta" ist mit "alpha" bezeichnet. Um das Doppelte dieses Winkels ist in Figur 1d die Nut 15 einseitig keilförmig erweitert.

Wenn die Zeichnung wiederum um die Linie 18 gefaltet gedacht wird, ist ersichtlich, daß die Feder 14' in die Nut 15' paßt. Weiterhin ist erkennbar, daß nach Drehung des Zwischenteils 5 (ausgehend von Figur 1c) um den Winkel "beta" die Stellung nach Figur 1e erhalten wird und daß nunmehr die Feder 14' in ihrer neuen Stellung nach Figur 1e ebenfalls in die Nut 15' nach Figur 1d hineinpaßt.

In Figur 1 ist weiterhin eine Möglichkeit zum Lösen der Konusverbindungen angegeben. Hierzu ist bei dem Zwischenteil 9 durch eine Bohrung 18 dafür gesorgt, daß nach dem Einsetzen des Endstückes 12 zwischen dessen Feder 14 und dem Zwischenteil 9 (genauer gesagt: dem oberen Wandungsteil der Bohrung 18) ein Zwischenraum bleibt. Dieser Zwischenraum ist von außen durch die Bohrung 18 für einen Keil 19 zugänglich, der in Richtung eines Pfeiles 20 eingetrieben werden kann, um den konischen Zapfen 11 wieder aus der Bohrung 10 hinauszudrücken. Der Keil 19 stützt sich dabei auf der Feder 14 ab, so daß beim Auseinandertreiben der Teile 9 und 12 die Konusflächen geschont werden.

Nicht in der Zeichnung dargestellt ist eine praktische Ergänzungsmöglichkeit, die darin besteht, daß die Prothesenteile mit einem Längskanal für einen vom Kopfteil 1 bis zum Endstück 12 reichenden Zuganker versehen sind. Dabei kann es sich um eine dünne durchgehende Schraube oder bevorzugt um eine biegsame Gewindestange handeln, welche die einzelnen Prothesenteile zusammenhält. Bei Verwendung einer Gewindestange kann diese mit einem Schneidwerkzeug, beispielsweise einer Zange, nach Bedarf auf die erforderliche Länge gekürzt werden. Ein solcher Zuganker ist an sich entbehrlich, da die Konusverbindungen sich bei Belastung setzen; er ist aber gleichfalls für die Handhabung während der Operation und zur Fixierung vor dem Beginn der eigentlichen Belastung durch das Körpergewicht des Patienten nützlich.

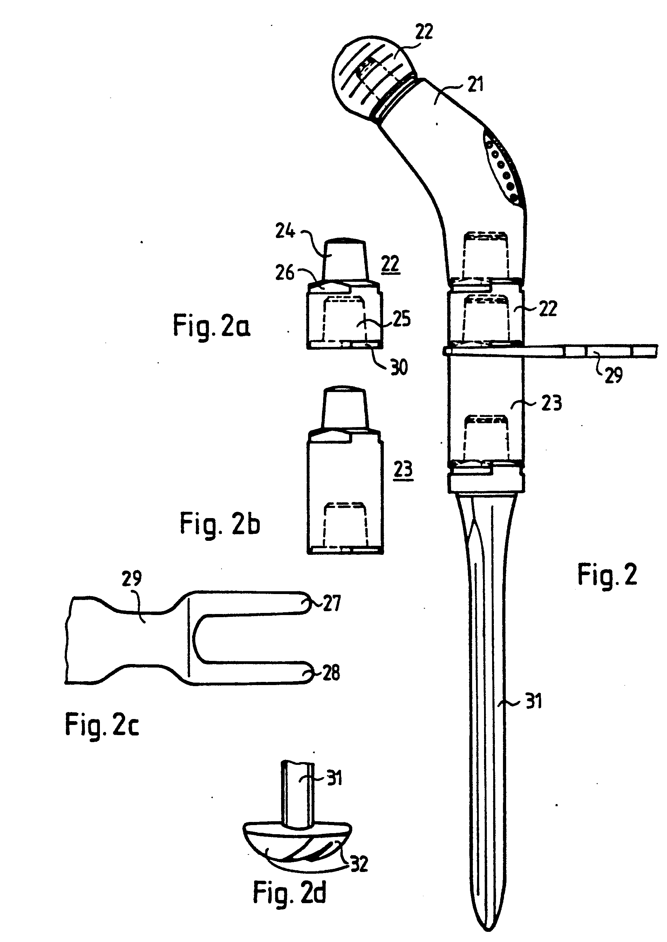

In den Figuren 2 bis 2c ist ein weiteres Ausführungsbeispiel der erfindungsgemäßen Resektionsprothese dargestellt. Ein gekrümmter Schaftteil 21 nimmt über einen Konus den Gelenkkopf 22 auf, wobei in der perspektivischen Darstellung der Konus - entsprechend bei den nachfolgenden Verbindungen - jeweils gestrichelt dargestellt ist.

An den gekrümmten Schaftteil 21 schließen sich zwei Bauteile unterschiedlicher Länge 22 und 23 an, welche in den Figuren 2a und 2b jeweils noch einmal separat dargestellt sind. Durch die Variation der Länge der Bauelemente lassen sich unterschiedliche Prothesenlängen realisieren. Bei dem in Figur 2a wiedergegebenen Element 22 ist ein Konus 24 erkennbar, welcher bezüglich seiner Abmessungen an die entsprechende, gestrichelt dargestellte, konusförmige Ausnehmung 25 angepaßt ist, welche in ihren Abmessungen einheitlich für das gesamte System verwendet wird. Das Element 22 weist an seiner dem Konus 24 zugewandten Seite zwei ebene Anfasungen auf, von denen nur die in der Zeichnung vorn liegende Anfasung 26 sichtbar ist. Die beiden Anfasungen liegen parallel zueinander und entsprechen in ihrem Abstand dem Abstand der Innenflächen von zwei keilförmigen Zinken 27 und 28 einer Gabel 29.

Diese Gabel 29 wird von der Seite her zwischen die Elemente getrieben, wie es in Figur 2 dargestellt ist. Der gabelförmige Keil 29 läßt sich somit leicht einsetzen und wird durch die beiden Anfasungen verkantungsfrei geführt. Ein Eindringen von Körperflüssigkeit in Innenbereiche der Prothese ist dabei nicht möglich, so daß sich die Prothesenteile später in jedem Falle leicht voneinander lösen lassen. Die keilförmigen Flächen des Teils 29 wirken dabei auf untere Begrenzungen der Anfasungen bzw. auf die Unterkante des jeweils obengelegenen Teils 1.

Der untere Bereich der eine konusförmige Aussparung aufweisenden Teile enthält eine zusätzliche Ausnehmung 30, welche so beschaffen ist, daß die neben der Ausnehmung verbleibenden Teile die Einzelteile der Prothese an ihrer den Konus 24 aufweisenden Seite im Bereich der Anfasungen 26 übergreifen und somit gegen ein Verdrehen sichern.

Falls eine Schaftkrümmung entsprechend der Ausführung als Links- bzw. Rechtsprothese erwünscht ist, sind die Flächen der Anfasung bzw. der diese übergreifenden Ausnehmungen entsprechend dem in Figuren 1c bis 1e dargestellten Ausführungsbeispiel auszugestalten.

Bei einem weiteren - in der Zeichnung nicht dargestellten - Ausführungsbeispiel ist der Endbereich des Schafts 31 derart ausgebildet, daß er in seinem unteren Bereich eine Aufnahme für ein künstliches Kniegelenk bildet oder er ist bereits selbst in einer Weise geformt, daß an diesem Teil die entsprechenden Abrollflächen vorgesehen sind. Eine derartige Ausführung einer Totalprothese ergibt eine hohe Stabilität und läßt einen erweiterten Anwendungsbereich für die erfindungsgemäße Prothese zu. Ein entsprechendes Element ist in der Zeichnung in Figur 2d dargestellt und bildet eine alternative Ausführung des Schaftendes 31 (hier mit 31' bezeichnet), wobei bei dieser Ausführung mit dem Schaftende 31' die Abrollflächen 32 eines künstlichen Kniegelenks fest verbunden sind.

Die Erfindung beschränkt sich in ihrer Ausführung nicht auf das vorstehend angegebene Beispiel. Vielmehr sind eine Vielzahl von Varianten denkbar, welche von der dargestellten Lösung auch bei grundsätzlich anders gearteten Ausführungen Gebrauch machen.

**Patentansprüche**

1. Bausatz für eine Resektionsprothese mit einem Kopfteil (1; 21) und einem Endteil (12; 31), von denen das eine Teil einen konischen Zapfen (11) und das andere Teil eine konische Bohrung (3) aufweist, wobei zwischen diesen beiden Teilen mindestens ein an Zapfen (11) und Bohrung (3) angepaßtes Zwischenteil (5, 9; 22, 23) vorgesehen ist, und die beim Zusammensetzen aneinanderstoßenden Flächen unterschiedlicher Teile durch aneinander angepaßte Ausformungen (14, 15) gegen Verdrehen sicherbar sind,
dadurch gekennzeichnet,
daß sich in aneinandergrenzenden Bereichen benachbarter Teile mindestens eine, in die im übrigen ebenmäßige Außenkontur mindestens eines der Teile eingelassene Ausnehmung (15, 26, 30) befindet, welche in zur Implantation ausgerichteter Position insgesamt einen Keil (19, 28) zum Trennen der beiden Teile aufnimmt, wobei an den beiden Teilen einander gegenüberliegende, jeweils im wesentlichen quer zur Konusachse verlaufende Anschlagflächen vorgesehen sind, welche jeweils die mittels des Keils (19, 28) ausgeübte, die beiden Teile trennende Kraft aufnehmen.

2. Bausatz nach Anspruch 1, dadurch gekennzeichnet, daß der Keil (28) gabelförmig und die beiden Zinken (27, 28) der Gahel jeweils für sich keilförmig ausgebildet sind, wobei die Ausnehmungen (26) an einander gegenüberliegenden äußeren Bereichen der Prothesenteile vorgesehen sind und der Abstand der beiden Gabelzinken (27, 28) an die Entfernung der beiden Ausnehmungen (26) angepaßt ist.

3. Bausatz nach Anspruch 2, quer zu den Auschlagflächen ansgerichtete dadurch gekennzeichnet, daß äußere Führungsflächen (26) vorgesehen sind, welche dem inneren Abstand von entsprechenden Flächen an der Innenseite der Gabelzinken (27, 28) entsprechen.

4. Bausatz nach einem der vorangehenden Ansprüche, dadurch gekennzeichnet, daß die Längsachse (13) des Zwischenteiles (5, 9) ähnlich dem natürlichen Femur gekrümmt ist und die Ausformungen (14, 15) derart gestaltet sind, daß sie in zwei Stellungen (Figur 1c und 1e) ineinander passen, in welchen die aneinanderstoßenden Teile um einen Winkel (beta) um ihre Längsachse (13) gedreht sind, der 180° beträgt oder dem natürlichen Winkel nahekommt, der zwischen den lokalen Krümmungsebenen der Längsachsen der beiden natürlichen langgestreckten Teile der Oberschenkelknochen eines Menschen gelegen ist.

5. Bausatz nach Anspruch 4, dadurch gekennzeichnet, daß die Ausformungen mit geringem Spiel ineinandergreifen.

6. Bausatz nach Anspruch 4 oder 5, dadurch gekennzeichnet, daß die Ausformungen als Nase und Ausnehmung auf beim Zusammensetzen einander gegenüberliegenden Flächen (16, 17) ausgebildet sind.

7. Bausatz nach Anspruch 6, dadurch gekennzeichnet, daß die Ausformungen als Feder (14) und Nut (15) ausgebildet sind.

8. Bausatz nach Anspruch 7, dadurch gekennzeichnet, daß die Feder (14') in einem stumpfen Winkel (beta) geknickt verläuft und die Nut (15') an einem ihrer Enden sich keilförmig erweitert.

9. Bausatz nach einem der vorangehenden Ansprüche, dadurch gekennzeichnet, daß je zwei Teile mit konischem Zapfen (11) bzw. konischer Bohrung (10) derart gestaltet sind, daß beim Zusammensetzen in der Konusachse (13) ein von außen für einen Keil (19) zugänglicher Zwischenraum verbleibt.

10. Bausatz nach einem der vorangehenden Ansprüche, dadurch gekennzeichnet, daß das Zwischenteil (5) einen Längskanal für einen vom Kopfteil (1) zum Endteil (12) verlaufenden Zuganker aufweist.

11. Bausatz nach einem der vorangehenden Ansprüche, dadurch gekennzeichnet, daß ein weiteres Endteil (31') ein Anschlußteil bzw. eine Abrollfläche (32) für ein künstliches Kniegelenk aufweist.

**Claims**

1. A modular resection prosthesis assembly having a head member (1, 2) and an end member (12; 31) of which the one member comprises a tapered pin (11) and the other member comprises a tapered bore (3), at least one intermediate member (59; 22, 23), adapted to pin (11) and bore (3), being provided between these two members, and the surfaces of different members which abut against one another on assembly being able to be secured against twisting by shaped portions (14,15) adapted to one another, characterised in that in adjacent regions of neighbouring members there is at least one recess (15, 26, 30) which is let into the otherwise plane external contour of at least one of the members and which, in the position aligned for implantation as a whole, receives a wedge (19, 28) to separate the two members, stop surfaces, situated opposite one another and each extending substantially transversely to the axis of the taper, being provided on the two members, each of which stop surfaces takes up the force exerted by means of the wedge (19, 28) and separating the two members.

2. A modular assembly as claimed in Claim 1, characterised in that the wedge (28) is made forkshaped and the two tines (27, 28) of the fork are each made wedge-shaped, the recesses (26) being provided at oppositely situated outer regions of the prosthesis members and the spacing of the two fork tines (27, 28) being adapted to the distance between the two recesses (26).

3. A modular assembly as claimed in Claim 2, characterised in that outer guide surfaces (26) are provided which are aligned transversely to the stop surfaces and which correspond to the inner spacing of corresponding surfaces on the inside of the fork tines (27, 28).

4. A modular assembly as claimed in any one of the preceding Claims, characterised in that the longitudinal axis (13) of the intermediate member (5, 9) is curved like the natural femur and the shaped portions (14, 15) are so formed that they fit one into the other in two positions (Figures 1c and 1e) in which the abutting members are turned about their longitudinal axis (13) through an angle (beta) which is 180° or is close to the natural angle between the local planes of curvature of the longitudinal axes of the two natural elongated portions of the thigh bone of a human being.

5. A modular assembly as claimed in Claim 4, characterised in that the shaped portions interlock with slight play.

6. A modular assembly as claimed in Claim 4 or 5, characterised in that the shaped portions are constructed in the form of projection and recess on surfaces (16, 17) which are situated opposite one another on assembly.

7. A modular assembly as claimed in Claim 6, characterised in that the shaped portions are constructed in the form of tongue (14) and groove (15).

8. A modular assembly as claimed in Claim 7, characterised in that the tongue (14') extends bent at an obtuse angle (beta) and the groove (15') widens out in the shape of a wedge at one of its ends.

9. A modular assembly as claimed in any one of the preceding Claims, characterised in that each of two members with tapered pin (11) or tapered bore (10) are formed in such a manner that on assembly in the taper axis, a gap remains which is accessible from the outside for a wedge (19).

10. A modular assembly as claimed in any one of the preceding Claims, characterised in that the intermediate member (5) comprises a longitudinal channel for a tie rod extending from the head member (1) to the end member (12).

11. A modular assembly as claimed in any one of the preceding Claims, characterised in that a further end member (31') comprises a connecting member or a rolling surface (32) for an artificial knee joint.

## Revendications

1. Assemblage modulaire pour une prothèse de résection, comprenant une pièce de tête (1; 21) et une pièce terminale (12; 31) dont l'une présente un tenon conique (11) et l'autre une cavité coniqué (3), ainsi qu'au moins une pièce intermédiaire (5, 9; 22, 23) disposée entre ces deux pièces et adaptée au tenon (11) et à la cavité (3), qui peuvent être bloquées en rotation relative par des formations (14, 15) mutuellement adaptées de surfaces venant s'appliquer l'une contre l'autre lors de l'opération d'assemblage, caractérisé en ce que

des zones adjacentes de pièces voisines présentent au moins, dans le contour extérieur par ailleurs plan d'au moins l'une des pièces, un évidement (15, 26, 30) qui, à la position d'implantation, reçoit un coin (19, 28) destiné à séparer les deux pièces, lesquelles sont pourvues de faces de butée situées l'une en face de l'autre, s'étendant chacuné à peu près transversalement à l'axe du cône et auxquelles est appliquée la force de séparation des deux plèces, exercée au moyen du coin (19, 28).

2. Assemblage selon la revendication 1, caractérisé en ce que le coin (28) est en forme de fourche et les deux dents (27, 28) de la fourche sont chacune réalisées sous la forme d'un coin, les évidements (26) étant prévus sur des zones extérieures situées l'une en face de l'autre des pièces de la prothèse et l'écartement des deux dents (27, 28) de la fourche étant adapté à la distance entre les deux évidements (26).

3. Assemblage selon la revendication 2, caractérisé par la prévision de surfaces de guidage extérieures (26), orientées transversalement aux surfaces de butée, qui correspondent à la distance intérieure de surfaces correspondantes sur le côté intérieur des dents (27, 28) de la fourche.

4. Assemblage selon une des revendications précédentes, caractérisé en ce que l'axe longitudinal (13) de la pièce intermédiaire (5, 9) est courbé à la façon du fémur naturel et que les formations (14, 15) pour le blocage en rotation sont réalisées de manière qu'elles s'ajustent l'une dans l'autre à deux positions (figures 1c et 1e), où les pièces appliquées l'une contre l'autre sont tournées autour de leur axe longitudinal (13) d'un angle (bêta) égal à 180° ou voisin de l'angle naturel compris entre les plans de courbure locaux des axes longitudinaux des deux parties oblongues naturelles des fémurs d'un être humain.

5. Assemblage selon la revendication 4, caractérisé en ce que les formations s'emboîtent mutuellement avec un faible jeu.

6. Assemblage selon la revendication 4 ou 5, caractérisé en ce que les formations sont réalisées comme une saillie et un évidement sur des surfaces (16, 17) s'appliquant l'une contre l'autre lors de l'opération d'assemblage.

7. Assemblage selon la revendication 6, caractérisé en ce que les formations sont réalisées comme une languette (14) et une rainure (15).

8. Assemblage selon la revendication 7, caractérisé en ce que la languette (14') possède une forme brisée définissant un angle obtus (bêta) et que la rainure (15') s'évase en forme de coin à ses extrémités.

9. Assemblage selon une des revendications précédentes, caractérisé en ce que chaque fois deux pièces, présentant l'une un tenon conique (11) et l'autre une cavité conique (10), sont conformées de manière qu'à la suite de la réunion sur l'axe du cône (13), il subsiste entre elles un intervalle dans lequel un coin (19) peut être enfoncé de l'extérieur.

10. Assemblage selon une des revendications précédentes, caractérisé en ce que la pièce intermédiaire (5) présente un canal longitudinal pour un tirant s'étendant depuis la pièce de tête (1) jusqu'à la pièce terminale (12).

11. Assemblage selon une des revendications précédentes, caractérisé en ce qu'une pièce terminale supplémentaire (31') présente ou forme une partie de raccordement ou une surface de roulement (32) pour une articulation de genou artificielle.

Fig.1c

Fig.1e

Fig.1a

Fig.1d

Fig.1b

Fig. 1

Fig.1f

Fig. 2a

Fig. 2b

Fig. 2c

Fig. 2d

Fig. 2